(19) Europäisches Patentamt

European Patent Office

Office européen des brevets

(11) **EP 4 650 025 A1**

(12) **EUROPEAN PATENT APPLICATION**

(43) Date of publication:
**19.11.2025  Bulletin 2025/47**

(21) Application number: **24176330.9**

(22) Date of filing: **16.05.2024**

(51) International Patent Classification (IPC):
**B01D 15/32** *(2006.01)*    **B01D 15/36** *(2006.01)*
**B01D 15/38** *(2006.01)*    **B01D 67/00** *(2006.01)*
**B01D 69/02** *(2006.01)*    **C07K 1/18** *(2006.01)*
**C12N 7/00** *(2006.01)*

(52) Cooperative Patent Classification (CPC):
**C07K 1/18; B01D 15/1896; B01D 15/363;
B01D 69/02; B01D 69/125; B01D 71/08;
B01D 71/82;** B01D 2323/30; B01D 2325/02;
B01D 2325/04; B01D 2325/14; B01D 2325/16;
C12N 2740/16051

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Sartorius Stedim Biotech GmbH
37079 Göttingen (DE)**

(72) Inventors:
• **Krause, André
  37079 Göttingen (DE)**
• **Thom, Volkmar
  37079 Göttingen (DE)**

• **Taft, Florian
  37079 Göttingen (DE)**
• **Schreiber, Tony
  37079 Göttingen (DE)**
• **Dauer, David-Raphael
  37079 Göttingen (DE)**
• **Cardoso, Sara
  37079 Göttingen (DE)**
• **Metze, Michael
  37079 Göttingen (DE)**

(74) Representative: **Müller-Boré & Partner
Patentanwälte PartG mbB
Friedenheimer Brücke 21
80639 München (DE)**

(54) **INTEGRAL POROUS POLYSACCHARIDE MEMBRANE AND METHOD OF PURIFYING BIOMOLECULES USING SAID MEMBRANE**

(57)    The present invention relates to a method of purifying biomolecules having a hydrodynamic diameter of at least 20 nm by using an integral porous polysaccharide membrane having chromatographically active centers. Moreover, the present invention relates to said integral porous polysaccharide membrane which has chromatographically active centers capable of binding a biomolecule having a hydrodynamic diameter of at least 20 nm, as well as a use of said membrane for purifying at least one type of biomolecules having a hydrodynamic diameter of at least 20 nm.

Figure 1

**Description**

The present invention relates to a method of purifying biomolecules having a hydrodynamic diameter of at least 20 nm by using an integral porous polysaccharide membrane having chromatographically active centers. Moreover, the present invention relates to said integral porous polysaccharide membrane which has chromatographically active centers capable of binding a biomolecule having a hydrodynamic diameter of at least 20 nm, as well as a use of said membrane for purifying at least one type of biomolecules having a hydrodynamic diameter of at least 20 nm.

Adsorption membranes are planar adsorbents being provided with pores passing from one side to the other side of the adsorbent. In this context, adsorbents are porous solid materials that are capable of selectively binding specific components of fluids passed over or through said material via functional groups at the surface (also called ligands). Target substances for binding as well as contaminants are typically single molecules, groups of molecules, complexes, or particles, which are often of biological origin (e.g., proteins).

Exemplary ligands (chromatographically active centers) which interact with such target substances or contaminants are ion exchangers, chelating ligands and heavy metal chelating ligands, sulfur-containing and hydrophobic ligands having specific configurations and chain lengths, reversed-phase systems, dye ligands, affinity ligands, amino acids, coenzymes, cofactors and analogs thereof, substrates and analogs thereof, endocrinic and exocrinic substances such as hormones and hormone-like agents, effectors and analogs thereof, enzyme substrates, enzyme inhibitors, fatty acids, fatty acid derivatives, conjugated fatty acids and analogs thereof, nucleic acids such as DNA, RNA and analogs and derivatives thereof (single, double, and/or multiple stranded), as well as peptide nucleic acids and derivatives thereof, viruses, virus-like particles, monomers and analogs and derivatives thereof, oligomers to polymers and analogs and derivatives thereof, linear and branched, substituted and non-substituted high-molecular carbohydrates, polymeric glycoconjugates such as heparin, amylose, cellulose, chitin, chitosan, and monomers, oligomers, derivatives, and analogs thereof, lignin and analogs and derivatives thereof, other biochemical/biological/chemical ligands such as oligo- and polypeptides (e.g., proteins and their oligomers), multimers, subunits and parts thereof, particularly lectins, antibodies, fusion proteins, haptens, enzymes and subunits and parts thereof, structural proteins, receptors and effectors and parts thereof, xenobiotics, pharmaceutics and pharmaceutical agents, alkaloids, antibiotics, biomimetics, etc.

One exemplary target of such adsorption membranes are biomolecules and particularly lentiviruses. Lentiviruses are capable of introducing larger connected genetic constructs stable into the genome of a target cell. Thus, lentiviruses are considered to be one of the most promising modalities in cell and gene therapy. In contrast to other gene vectors such as mRNA, plasmid DNA (pDNA), or adeno-associated viruses (AAVs), lentiviruses are enveloped by a sensitive lipid double layer. Therefore, development of new strategies of purifying lentiviruses requires exceptionally mild process parameters in order to maintain their structural and functional integrity leaving small room for optimizing said parameters (e.g., pH $7 \pm 1$ and temperature $\leq 4$ °C) besides ionic strength (which also becomes critical at moderate ionic strength of e.g., a NaCl concentration of 400 to 1000 mM). Due to their pI (isoelectric point) of about 6, lentiviruses are commonly purified using anion exchangers. Even further, due to the sensitivity of lentiviruses and due to themselves being viruses, downstream sterilization treatments are not or nearly not possible.

However, commercially available adsorption membranes, i.e., their stationary phases, are not optimized for purifying biomolecules such as sensitive and large (50 to 300 nm hydrodynamic diameter) biomolecules. Typically, the stationary phases of such commercially available adsorption materials or membranes are based on resins, monoliths, fibers, or polymer membranes. Particularly for resins, but also for other stationary phases, the pore size distribution results in part of the volume of the stationary phase which cannot be accessed by large particles (or may only be accessed extremely slowly due to diffusive resistance). Moreover, particularly for monoliths and packed fibers, the porosity of the stationary phase is low resulting in low binding capacity and reduced permeability connected to increased material consumption. Even further, in the particular cases of resins and packed fibers, the stationary phase is built from a plurality of single units (i.e., resin beads and fibers) therefore causing increased effort in packing and possible contamination of the eluted, purified product with the particles of the stationary phase. As can be seen, the commercially available adsorption membranes are not optimized for achieving a maximal inner surface area for binding large biomolecules (high binding capacity) while also maintaining a high permeability. That is, the binding capacity is low connected to a typical pressure drop during chromatography resulting in decreased productivity.

Moreover, known and commercially available adsorption membranes typically have high ligand densities, i.e., 100 μmol/mL of membrane volume or more. Such high ligand density causes strong bonds or attractive interactions to be formed between the stationary phase and the target molecule which is prone to impair the structural and functional integrity of unstable target molecules. Further, to elute the target molecules from such stationary phase elution liquids having e.g. high ionic strength are necessary which increases the probability of damage to sensitive target molecules even more. Finally, the high ligand density results in increased unspecific binding of contaminants due to free binding sites, thus, resulting in lower selectivity and worse purification results.

Accordingly, in view of the above, the technical problem underlying the present invention is to provide a method of purifying biomolecules having a hydrodynamic diameter of at least 20 nm, the method achieving a high yield and selectivity against contaminants as well as yielding a highly concentrated purified suspension of said biomolecules, while it is also possible to maintain mild elution parameters in order to avoid deactivation/degradation of said biomolecules. A further technical problem underlying the present invention is to provide an integral porous membrane which is suitable for the above method.

The solution to the above technical problems is provided by the embodiments characterized in the claims.

Method of purifying biomolecules having a hydrodynamic diameter of at least 20 nm

Accordingly, the present invention provides a method of purifying biomolecules having a hydrodynamic diameter of at least 20 nm, comprising

a) providing an integral porous polysaccharide membrane and a suspension comprising at least one type of biomolecules having a hydrodynamic diameter of at least 20 nm,
b) bringing the suspension comprising at least one type of biomolecules into contact with the integral porous polysaccharide membrane to bind the at least one type of biomolecules to the integral porous polysaccharide membrane,
c) optionally washing the integral porous polysaccharide membrane having bound the at least one type of biomolecules with at least one washing liquid, and
d) eluting the at least one type of biomolecules from the integral porous polysaccharide membrane by passing an eluting liquid through the integral porous polysaccharide membrane to obtain a purified suspension comprising the at least one type of biomolecules;

wherein the integral porous polysaccharide membrane has a first main surface, a second main surface, and membrane bridges which are a solid polymer bulk material surrounding and delimiting the pores of the membrane,
wherein the average pore diameter of the membrane pores in the porous polysaccharide membrane is from 0.45 to 10.0 $\mu$m,
wherein the convective porosity of the integral porous polysaccharide membrane is at least 60%,
wherein the integral porous polysaccharide membrane has chromatographically active centers capable of binding a biomolecule having a hydrodynamic diameter of at least 20 nm attached to its inner and outer surfaces, and
wherein an amount of the chromatographically active centers per volume of the membrane is from 2 to 12 $\mu$mol/mL and
an inner surface area of the integral porous polysaccharide membrane is from 1 to 4 m$^2$/g.

In this context, the herein described method of purifying biomolecules having a hydrodynamic diameter of at least 20 nm corresponds to a bind-and-elute chromatographic method. That is, such method is characterized by including a step of binding at least a part of the components, preferably the target components, of a feed suspension or solution to an adsorptive material followed by a separate step of eluting the bound components from the adsorptive material. Typically, in between said binding and eluting steps, a washing step is incorporated which elutes components of the suspension or solution which are not bound to the adsorptive material, such as contaminants.

Further, the term "biomolecule" as used herein is not limited to specific molecules and relates to any biomolecule having a hydrodynamic diameter of at least 20 nm. Examples of such large biomolecules include proteins, protein complexes, nucleic acids, carbohydrates and derivatives thereof, lipid double layer-enveloped entities such as liposomes, cell organelles, viruses, virus-like particles (VLPs), extracellular vesicles, exosomes, and bacteria.

Here, it is to be noted that the term "biomolecules" relates to target molecules of the herein described purification method, i.e., said term does not include contaminants that are to be separated from the biomolecules. In this context, the term "contaminants" relates to components and, specifically, proteinaceous and nucleic acid components included in a cell lysate (i.e., cell debris), i.e., a suspension obtained by cell lysis, that are detected by e.g., Bradford assay or that are stained with specific dyes (e.g., Coomassie Brilliant Blue G 250). In view of the above definition of biomolecules, the contaminants, typically, are not enveloped by a lipid double layer and have a hydrodynamic diameter of less than 20 nm.

The biomolecules that can be purified with the herein described method have a hydrodynamic diameter of at least 20 nm. Preferred biomolecules which can be purified with the herein described method have a hydrodynamic diameter of at least 50 nm and more preferred biomolecules have a hydrodynamic diameter of at least 70 nm.

In this context, the term "suspension" is used in the herein described invention, since biomolecules having a hydrodynamic diameter of at least 20 nm are commonly recognized as being suspended in a liquid as nanoparticles.

Moreover, the term "integral porous polysaccharide membrane" (hereinafter sometimes referred to as "membrane") relates to a membrane comprising, preferably as a main constituent, a polysaccharide, for example, being comprised of more than 50% of a polysaccharide, which has a first main surface, a second main surface, and membrane bridges which are a solid polysaccharide bulk material surrounding and delimiting the pores of the membrane, and which is made from a single piece (the single piece being (a) the material of the membrane alone, or (b) in case of an optional support structure as described later, the membrane material intermingled with the support structure). That is, the herein described membrane comprises at least two phases: a solid polymer bulk phase forming the membrane bridges and at least one second phase between the membrane bridges forming pores.

The polysaccharide is not particularly limited and may be any hydrophilic polysaccharide, due to the low unspecific binding of biomolecules of hydrophilic materials, i.e., having a binding capacity of bovine serum albumin (BSA) of less than 0.2 mg/ml membrane volume. In view of an especially low unspecific binding of biomolecules, preferred examples thereof include cellulose, such as regenerated cellulose and crosslinked cellulose, agarose, and crosslinked agarose.

In the integral porous polysaccharide membrane according to the present invention, the average pore diameter of the membrane pores in the porous polysaccharide membrane is from 0.45 to 10.0 $\mu$m, preferably of from 0.50 to 7.0 $\mu$m, more preferably of from 0.60 to 4.0 $\mu$m, even more preferably of from 0.70 to 2.50 $\mu$m, still more preferably of from 0.80 to 1.50 $\mu$m, and most preferably of from 1.00 to 1.20 $\mu$m. In this context, the average pore diameter is determined using a "Capillary Flow Porometry Test" as described e.g., in the manual of the "Capillary Flow Porometer 6.0" (CAPWIN Software System, Porous Materials Inc.). A membrane having the above average pore diameter exhibits a high flowrate in the herein described method while also preventing fouling and/or deactivation of the membrane due to contaminations.

Moreover, the convective porosity of the membrane is at least 60%. In this context, the convective porosity is the volume of the convective pores ($V_{\text{convective pores}}$) divided by the whole volume of the complete membrane ($V_{\text{complete membrane}}$), which is the sum of $V_{\text{convective pores}}$ and the volume of the polysaccharide membrane material ($V_{\text{membrane material}}$) including the optional support (which is described later). In this context, the volume of the convective pores of the membrane may be determined by inverse size exclusion chromatography as known by a person skilled in the art and shown exemplarily in a modified form in Journal of Chromatography A - J CHROMATOGR A. 731. 27-40. 10.1016/0021-9673(95)01197-8, or in the case of neutral and negatively charged membranes using the dextran blue method according to e.g., Example 14 on pages 39 to 42 of WO 2009/127285 A1, and the volume of the complete membrane may be determined by measuring the geometric thickness and dimensions and calculating the volume depending on the shape of the membrane, e.g., of a cylinder.

$$convective\ porosity = \frac{V_{convective\ pores}}{V_{complete\ membrane}}$$

with

$$V_{complete\ membrane} = V_{convective\ pores} + V_{membrane\ material}$$

The "membrane material" as used above is the polysaccharide material and the (optional) support, and thus the volume of the membrane material is the sum of the volume of the polysaccharide material and volume of the (optional) support.

In preferred embodiments, the convective porosity is at least 65%, more preferred at least 70%, even more preferred at least 75%, still more preferred at least 80%, and most preferred at least 85%. Further, the convective porosity may be at most 90%. If the convective porosity is at least 60%, an excellent balance between flowrate of a solution through the membrane and a high inner surface area is achieved. By providing a convective porosity of at most 90%, the stability of the membrane can be ensured, which may deteriorate at very high convective porosities.

Further, the integral porous polysaccharide membrane has chromatographically active centers capable of binding a biomolecule having a hydrodynamic diameter of at least 20 nm attached to its inner and outer surfaces.

In this context, the term "chromatographically active center" (also called "ligand") relates to a moiety bound to the polysaccharide material, which is able to interact and/or bind with biomolecules of the suspension/mobile phase that is brought into contact with the membrane.

With regard to ligands/chromatographically active centers which interact with the biomolecules of the mobile phase, mention can be made, by way of example, of ion exchangers, chelating agents and heavy metal chelates, thiophilic and hydrophobic ligands of various chain lengths and configurations, reversed-phase systems, dye ligands, affinity ligands, amino acids, coenzymes, cofactors and their analogs, substrates and their analogs, endocrine and exocrine substances, such as hormones and active ingredients acting like hormones, effectors and their analogs, enzyme substrates, enzyme inhibitors and their analogs, fatty acids, fatty acid derivatives, conjugated fatty acids and their analogs, nucleic acids, such as DNA, RNA, and their analogs and derivatives (single-, double-, and/or multistranded), and also peptide nucleic acids and their derivatives, viruses, virus particles, monomers and their analogs and derivatives, oligomers to polymers and their analogs and derivatives, high-molecular weight carbohydrates, which can be linear or branched, unsubstituted or substituted, polymeric glycoconjugates, such as heparin, amylose, chitin, chitosan, and their monomers and oligomers and derivatives and analogs thereof, lignin and its derivatives and analogs, other biochemical ligands, such as oligopeptides and polypeptides, e.g., proteins and their oligomers, multimers, subunits and also parts thereof, more particularly lectins, antibodies, fusion proteins, haptens, enzymes, and subunits and also parts thereof, structural proteins, receptors and effectors and also parts thereof, and in addition xenobiotics, pharmaceuticals and pharmaceutical active ingredients, alkaloids, antibiotics, biomimetics, etc. That is, the herein described ligands/chromatographically active centers interact and/or bind with biomolecules via ionic, hydrophobic, electron donor acceptor, hydrogen bonding, $\pi$-$\pi$ interactions, or combinations thereof (i.e., multi modal moieties). Further, the above ligands/chromatographically active centers, particularly ion exchangers, chelating agents and heavy metal chelates, thiophilic and hydrophobic ligands of various chain lengths and configurations, and reversed-phase systems, may also be combined for mixed mode chromatography. Preferably, the ligands which are capable of interacting and/or binding with biomolecules interact via anionic, cationic, hydrophobic interactions or a combination thereof.

According to the present invention, an amount of the chromatographically active centers per volume unit of the membrane is from 2 to 12 $\mu$mol/mL, preferably from 3 to 11.5 $\mu$mol/mL, more preferably from 4 to 11 $\mu$mol/mL, even more preferably from 6 to 10.5 $\mu$mol/mL, and most preferably from 8 to 10 $\mu$mol/mL. In this context, the amount of the chromatographically active centers in the membrane is the amount of said centers/ligands in the membrane volume including the membrane material (i.e., comprising the polysaccharide material and the optional support described later) and the pores. Further, the above amount is given with the unit of $\mu$mol/mL, which can be converted to $\mu$mol/g using the density (e.g., a membrane according to the present invention having a density of 0.27 g/mL has an amount of ligands of from 7.45 to 44.67 $\mu$mol/g).

The amount of ligand may be determined depending on the type of ligand. That is, the amount of anion-exchange ligands, which represents a preferred embodiment as described later, (in the so-called Q or D membranes) may be determined as follows:

A membrane having a volume of 0.08 mL is loaded with 50 mL of a 0.1 M potassium phosphate solution in water being subjected to reverse osmosis (ROW) at pH 7.0. Then, said membrane is washed with 50 mL ROW with exclusion of salts and eluted using 15 g of a 10% (w/w) $Na_2SO_4$ solution in ROW. An aliquot of 5 g of the eluate are mixed with 5 g of a reactant solution consisting of 0.5% (w/w) ammonium heptamolybdate tetrahydrate, 10% (w/w) sulfuric acid $H_2SO_4$, and 2% (w/w) L(+)-ascorbic acid in ROW followed by incubation for at least 15 min at 65 to 75 °C. Subsequently, the phosphate concentration is determined photometrically at a wavelength of 825 nm.

Similarly, the amount of cation-exchange ligands, which represents another preferred embodiment as described later, (in the so-called S membranes) may be determined as follows:

A membrane having a volume of 0.08 mL is loaded with 50 mL of a 2% (w/w) ammonium sulfate $(NH_4)_2SO_4$ solution in ROW at pH 7.0. Then, said membrane is washed with 50 mL ROW with exclusion of salts and eluted using 15 g of a 10% (w/w) $Na_2SO_4$ solution in ROW. An aliquot of 4 g of the eluate are mixed with 4 g of a reactant solution consisting of 3.029% (w/w) sodium salicylate and 0.015% (w/w) sodium nitroprusside in ROW and 2 g of an aqueous solution of 1% (w/w) sodium dihydrogen phosphate, 10.8% (w/w) sodium hypochlorite (0.7 M) and 6% (w/w) sodium hydroxide (18.9 M) followed by incubation for at least 20 min at 40 °C. Subsequently, the ammonium concentration is determined photometrically at a wavelength of 660 nm.

In the above methods for photometrically determining the ligand amount, in order to determine quantitative amounts, first, a linear standard curve has to be measured with solutions having known amounts of either of phosphate or ammonium ions at the respective wavelengths.

Further, according to the present invention, an inner surface area of the integral porous polysaccharide membrane is from 1 to 4 $m^2$/g. Preferably, the inner surface area of the membrane is from 1.5 to 3.5 $m^2$/g and more preferably from 2 to 3 $m^2$/g. In this context, the inner surface is normalized to the weight of the measured membrane.

That is, the integral porous polysaccharide membrane has a high inner surface area and, thus, a high binding capacity of biomolecules. In this context, the inner surface area may be determined by the BET method known to a skilled person and as is described e.g., in Atkins, "Physikalische Chemie", 3rd edition, pp. 934-936, for instance using a Micromeritics Gemini VII and using $N_2$ as measuring gas.

Moreover, the method of the present invention comprises at least steps a), b), and d), and optionally step c):

a) providing an integral porous polysaccharide membrane and a suspension comprising at least one type of biomolecules having a hydrodynamic diameter of at least 20 nm,
b) bringing the suspension comprising at least one type of biomolecules into contact with the integral porous polysaccharide membrane to bind the at least one type of biomolecules to the integral porous polysaccharide membrane,
c) optionally washing the integral porous polysaccharide membrane having bound the at least one type of biomolecules with at least one washing liquid, and
d) eluting the at least one type of biomolecules from the integral porous polysaccharide membrane by passing an eluting liquid through the integral porous polysaccharide membrane to obtain a purified suspension comprising the at least one type of biomolecules.

In step a), the above-described integral porous polysaccharide membrane and a suspension comprising at least one type of biomolecules having a hydrodynamic diameter of at least 20 nm are provided.

In step b), the above suspension is brought into contact with the integral porous polysaccharide membrane and the at least one type of biomolecules is bound to the membrane. Said step of bringing the suspension and the membrane into contact is not particularly limited and may be any known method of bringing such suspension and membrane into contact. For instance, the suspension may be passed through the membrane in a thickness direction of the membrane, i.e., a normal flow chromatography is conducted, or the suspension may be flowed over the membrane, i.e., a tangential flow chromatography is conducted. In step b), specific components of the suspension are bound to the membrane depending on the size of said components in comparison to the average pore diameter of the membrane and on the type of attached chromatographically active centers/ligands as well as the type of component of the suspension. In other words, the herein described membrane is optimized for specifically binding, with its chromatographically active centers, biomolecules of interest while letting contaminants either pass or filtering them off by size exclusion.

In optional step c), the membrane including the bound biomolecules is washed with at least one washing liquid. Said step achieves further purification of the biomolecules, since left over contaminants are washed away. Such washing step and suitable washing liquids are well-known to those skilled in the art.

Finally, in step d), the bound biomolecules are eluted from the membrane using an eluting liquid. Since possible contaminants have either been excluded or washed away in steps b) and the optional step c), a purified suspension of biomolecules is obtained having a reduced amount of contaminants. Such eluting step and suitable eluting liquids are well-known to those skilled in the art.

Due to the specific integral porous polysaccharide membrane, a method can be provided for purifying biomolecules having a hydrodynamic diameter of at least 20 nm, the method achieving a high yield and selectivity against contaminants as well as yielding a highly concentrated purified suspension of said biomolecules, while it is also possible to maintain mild elution parameters in order to avoid deactivation/degradation of said biomolecules.

According to a preferred embodiment of the method according to the present invention, examples of chromatographically active centers are ion exchangers, i.e., anionic and cationic groups such as, e.g., sulfonic acid groups, phosphoric acid groups, or carboxylic acid groups, and, e.g., ammonium groups. Said ion-exchange chromatographically active centers may also be used in combination in mixed mode chromatography. Introduction of anionic ligands (cation exchangers) enables a respective membrane to bind cationic components of a suspension/solution, while allowing anionic substances to pass. On the other hand, introduction of cationic ligands (anion exchangers) enables a respective membrane to bind anionic components of a suspension/solution, while allowing anionic substances to pass.

In this context, the term "ion-exchange" chromatographically active center relates to the above-described moieties which are capable of interacting and/or binding with biomolecules via ionic interactions. The herein described ion-exchange ligands are weak ion-exchange ligands, i.e., their charge is dependent on pH, or, in other words, they are pH sensitive. In contrast, strong ion-exchange ligands are charged independent of the pH. Examples of weak anion exchangers are $NH_4^+$ having a pKa value of 9.24, $HNMe_3^+$ having a pKa value of 9.80, $HNEt_3^+$ having a pKa value of 10.76, and $H_2NEt_2^+$ having a pKa value of 10.98. It should be noted that the afore-mentioned nitrogen-based anion exchangers are ions which are bound to the membrane via linking-groups.

According to a preferred embodiment of the method according to the present invention, the chromatographically active centers/ligands are anion-exchange chromatographically active centers/ligands.

Further, preferably the ion-exchange and specifically anion-exchange chromatographically active centers are one or more selected from primary, secondary, tertiary, and quaternary ammonium residues. Specific examples thereof include trimethyl ammonium residue, triethyl ammonium residue, dimethyl ammonium residue, diethyl ammonium residue, (2-aminoethyl)ammonium residue, (2-(N,N-diethylamino)ethyl)-N'-ethylammonium residue, (5-amino-3-azapentane)ammonium residue, (2-methylamino ethyl)-N'-methylammonium residue, (2-ethylamino ethyl)-N'-ethylammonium residue, (2-methylamino ethyl)ammonium residue, (3-aminopropyl)ammonium residue, (3-amino-2-hydroxypropyl)ammonium residue, (3-amino-2-methylpropyl)ammonium residue, and combinations thereof.

Moreover, in a specific embodiment, the at least one type of biomolecules having a hydrodynamic diameter of at least 20 nm are lipid double layer-enveloped entities and the chromatographically active centers are ion-exchange chromatographically active centers. That is, lipid double layer-enveloped entities preferably interact with ion-exchange ligands and, thus, can be specifically bound in step b) of the herein described method. In particular, biological membranes are often negatively charged rendering anion-exchange ligands to be most preferred. As described above, such lipid double layer-enveloped entities are not particularly limited and include e.g., liposomes, cell organelles, viruses, virus-like particles (VLPs), extracellular vesicles, and exosomes. In a specific embodiment, the at least one type of biomolecules are lentiviruses.

It is to be noted that, in principle, contaminants may also be enveloped by a lipid double layer. In such cases, it is possible to optimize the chromatographically active centers/ligands to bind specific lipid double layer-enveloped biomolecules of interest while letting lipid double layer-enveloped contaminants pass. This may be achieved e.g., due to specific lipid double layer compositions of both species or by using multi modal ligands that bind via ionic interactions and other interactions specific to the lipid double layer of the target biomolecules.

In a further preferred embodiment of the method according to the present invention, the chromatographically active centers/ligands are multi model moieties. Here, a respective membrane comprises at least two different and cooperative binding sites that interact with the biomolecules.

In one specific embodiment, the membrane comprises two or more functional groups that are bound to the same ligand. Any of the above-described functional groups can be used as multi modal moieties, e.g., the combination of quaternary ammonium groups with phenyl groups.

For instance, the ligands may be connected to the membrane via one of said functional groups, e.g., via amines, thus, yielding quaternary ammonium binding sites. Moreover, the two or more functional groups may be separated via a carbohydrate spacer having e.g., 1 to 6, preferably 1 to 3, more preferably 1 or 2 carbon atoms. In a further specific embodiment, the ligands are selected from the group consisting of N-benzyl-N-methylethanolamine, N,N-dimethylbenzylamine, 2-aminobenzimidazole, and thiomicamine.

Alternatively, the membrane may comprise two or more functional groups bound to separate ligands.

In a further preferred embodiment of the method according to the present invention, the chromatographically active centers/ligands are affinity ligands such as p-aminobenzamidine, biomimetic ligands, and/or proteins.

In a further preferred embodiment of the method according to the present invention, the chromatographically active centers/ligands are metal chelate ligands.

In a further preferred embodiment of the method according to the present invention, the chromatographically active centers/ligands are hydrophobic ligands. Said hydrophobic ligands are preferably selected from the group consisting of $C_1$-$C_{20}$ alkyl groups, $C_6$-$C_{25}$ aryl groups, $C_7$-$C_{25}$ aryl alkyl groups, and derivatives thereof, and/or -$[(CH_2)_m$-O-$]_n$-R, wherein m is 2 or 3 and n is an integer of more than 1 and wherein R is H or a $C_1$-$C_5$ alkyl group.

In a further preferred embodiment of the method according to the present invention, the chromatographically active centers/ligands are reactive ligands selected from the group consisting of epoxy, aldehyde, azolactone, N-hydroxysuccinimide, and/or carbodiimide groups.

In a further preferred embodiment of the method according to the present invention, the chromatographically active centers/ligands are catalytically active ligands such as for example acids, bases, immobilized metals, or enzymes.

It is further preferred that the chromatographically active centers/ligands are bound to the membrane via an oligomeric or polymeric spacer, or, even more preferable, bound directly to the membrane.

Moreover, it is preferred that the integral porous polysaccharide membrane has a thickness of from 50 to 1000 μm. More preferably, the membrane has a thickness of from 100 to 500 μm, even more preferably of from 150 to 300 μm, and most preferably of from 210 to 260 μm. Providing the herein described membrane in said thickness range achieves high mechanical stability of the membrane in the herein described purification method.

According to a specific embodiment of the present invention, the porous polymer membrane further comprises a support structure. In this context, the optional support structure is a part of the membrane and not a separate entity. The support structure is not particularly limited. Therefore, it is possible to use any support layer known to the skilled person from the prior art. For example, the support layer may be a nonwoven web, a woven fabric, or an open microfilter membrane. Examples of nonwoven webs are polyolefin nonwovens, such as, for example, PP/PE core-shell nonwovens and polyester nonwovens. The support layer is preferably a polyolefin nonwoven or a polyolefin membrane. Providing a support layer in the herein described membrane further improves mechanical stability of the membrane while maintaining its excellent properties regarding binding of substances, flowrate of a mobile phase, and inner surface area.

Moreover, in a specific embodiment of the method according to the present invention, an integral porous polysaccharide membrane is provided, wherein the average pore diameter of the membrane pores in the porous polysaccharide membrane is from 0.45 to 10.0 μm, the thickness of the porous polysaccharide membrane is from 150 to 300 μm, and the porosity of the porous polysaccharide membrane is at least 65%. Providing said integral porous polysaccharide membrane having the specific average pore diameter, specific thickness, and specific porosity achieves a chromatographic material having a high binding capacity while a high flowrate of a fluid passing over or through the membrane can be maintained.

In a preferred embodiment, the integral porous polysaccharide membrane has a permeability of from 20 to 500 mD, preferably of from 40 to 300 mD, and more preferably of from 80 to 200 mD. If the permeability of the membrane is in the above ranges, the membrane is characterized by a good balance between flowrate and binding capacity of biomolecules. Said balance is particularly excellent within the above preferred ranges.

The permeability may be determined by the following formula as is described e.g., in Ley et al. Journal of Membrane Science 564, 2018, 543-551 (page 545):

$$P[mD] = \left( v \left[ \frac{cm^3}{cm^2 \cdot s} \right] * \mu[cP] * \frac{\Delta x[cm]}{\Delta p[atm]} \right) * 1000$$

wherein P represents the permeability, v represents the area-related flowrate, $\mu$ represents the viscosity of water, $\Delta x$ represents the thickness of the layer through which the flow passes, and $\Delta p$ represents the pressure difference (cf. Darcy laws). In this context, the thickness of the layer through which the flow passes may be determined using a conventional thickness meter such as J50C (Käfer, Germany). The above determination of the permeability is conducted using a 47 die-cut membrane mounted in a housing equipped with a water tank, applying 100 mbar of pressure and measuring the time, which 100 mL water require to pass through the membrane.

In a further preferred embodiment of the present invention, a step of sterilizing may be conducted prior to step b). Sterilization may be achieved by providing a pre-sterilized integral porous polysaccharide membrane in step a). Further, sterilization may be achieved by gamma-irradiating the integral porous polysaccharide membrane prior to step b) using an irradiation intensity of from 10.0 to 100 kGy, preferably of from 25.0 to 50.0 kGy. Conducting step b) in a sterile environment, i.e., using a sterile membrane, avoids contamination of the biomolecules to be purified.

Moreover, the method according to the present invention preferably comprises an equilibration step prior to step b) of equilibrating the integral porous polysaccharide membrane with an equilibration liquid, which may correspond to the same or a similar composition as the suspension comprising at least one type of biomolecules, in order to prepare the membrane for application of said suspension. For instance, such equilibration step may be conducted for 4 min, e.g., by passing the equilibration liquid over the membrane with a flowrate of 5 membrane volumes per minute (MV/min) over 20 membrane volumes (MV).

Further, the pH of the suspension comprising the at least one type of biomolecules and/or the pH of the purified suspension comprising the at least one type of biomolecules is not particularly limited and depends on the biomolecules to be purified. However, in view of typical biomolecules of interest, preferably, the pH of the suspension comprising the at least one type of biomolecules is from 6.0 to 9.0, and/or the pH of the purified suspension comprising the at least one type of biomolecules is from 6.0 to 9.0. More preferably, the pH of either or both of the suspension comprising the at least one type of biomolecules and the purified suspension comprising the at least one type of biomolecules is from 7.0 to 8.0. By having the above pH values, the biomolecules to be purified remain stable, i.e., they remain active for further applications in case of e.g., viruses such as lentiviruses.

In a preferred embodiment of the present invention, the conductivity of the suspension of the at least one type of biomolecules is at most 15.0 mS/cm, and/or the conductivity of the at least one washing liquid is from 5.0 to 15.0 mS/cm, and/or the conductivity of the eluting liquid is at most 85 mS/cm. In particular, the conductivity is a measure for ionic strength which is one main factor determining the stability of most biomolecules. That is, most biomolecules require a specific conductivity/ionic strength of a suspension in order to remain stable and active such as a low conductivity/ionic strength. Therefore, the conductivity of the suspension of the at least one type of biomolecules may be at most 15.0 mS/cm, preferably at most 10.0 mS/cm, which achieves a high stability of the biomolecules in the feed suspension. Further, the conductivity of the at least one washing liquid may be from 5.0 to 15.0 mS/cm, preferably from 5.0 to 10.0 mS/cm, which ensures stability of the biomolecules during the optional washing step c) while also ensuring that contaminants are washed away. Even further, the conductivity of the eluting liquid may be at most 85 mS/cm, preferably from 40.0 to 75 mS/cm, and more preferably from 55 to 65 mS/cm, which ensures a high enough conductivity to elute the biomolecules while enabling them to remain stable and active during and after elution, and which further achieves a high yield of biomolecules being eluted from the membrane. In a specific embodiment, wherein the biomolecules are lentiviruses, the conductivity of the eluting liquid may be from 55 to 63 mS/cm, since said viruses are especially sensitive to high conductivity/ionic strength.

It is further preferred in the method according to the present invention that the temperature in steps b) to d) is from 0 to 37 °C. More preferably, the temperature in said steps is from 2 to 25 °C, and most preferably from 4 to 10 °C. Said temperatures may be applied to steps b) to d) as well as to step a), and even to each step individually. Ensuring the above temperatures ensures a high stability of the biomolecules to be purified. In particular, the upper limit of the temperature is at most 37 °C at which temperature most biomolecules remain stable and active. Moreover, the lower limit is set to the melting temperature of water or slightly higher to ensure a liquid medium having suitable properties for chromatography such as suitable viscosity for chromatographic methods.

In view of the above parameters of temperature and conductivity, it should be noted that at least some biomolecules are especially sensitive to deactivation at environments not native to said biomolecules, which is often the case in chromatography. Thus, in a preferred embodiment of the method according to the present invention, the exposure time to critical conditions, i.e., in steps b) to d), such as high salt content or high temperature is minimized. For instance, the exposure time to critical conditions may be at most 8 min, preferably at most 6 min, more preferably at most 5 min, even more preferably at most 4 min, and most preferably at most 3 min. Moreover, a lower limit of the exposure time to critical conditions may be 10 s in order to ensure enough time for binding, optional washing, and eluting of the biomolecules. In this context, the specific described exposure times to critical conditions may be achieved by optimizing the times of each of steps b) to d). That is, step b) may be conducted for e.g., 480 s, optional step c) may be conducted for e.g., 240 s, and step d) may be conducted for e.g., 240 s. In particular, the exposure time may be optimized by setting a suitable flowrate in each step such as e.g., 5 MV/min. Moreover, it is preferred to conduct a further step e) of fast substitution of the eluting liquid by a storage liquid suspending the purified biomolecules after eluting the biomolecules from the membrane. That is, the eluting liquid is preferably substituted by a more native buffer having e.g., a lower conductivity and/or lower temperature than the eluting liquid in order to enable storage of the purified biomolecules, wherein said biomolecules remain stable and active.

In particular and in view of the above considerations regarding stability of the biomolecules, the following conditions may be preferably met:

First, the biomolecule concentration in the suspension comprising at least one type of biomolecules is preferably set to at most $5.0 \times 10^{10}$ particles/mL, more preferably to at most $4.0 \times 10^{10}$ particles/mL, even more preferably to at most $3.0 \times 10^{10}$ particles/mL, and most preferably to at most $2.0 \times 10^{10}$ particles/mL. Moreover, the suspension comprising the at least one type of biomolecules may be an aqueous buffered suspension, wherein the buffer is not particularly limited and any known buffer system suitable for working with biomolecules may be used, e.g., Tris and HEPES buffers.

Further, with reference to the above-mentioned suitable flowrate of 5 MV/min, optimized exposure times to critical conditions may be achieved by specific volumes of liquid passed through the membrane in each step such as e.g., 40 MV in step b) and 20 MV in optional step c). Moreover, the eluting step d) may be conducted as a gradient elution e.g., over 20 MV from 0% to 75% of eluting liquid (i.e., from 100% to 25% of washing liquid), wherein the eluting liquid contains, for instance, 1 M NaCl, yielding a final NaCl concentration of 750 mM. Further, said gradient elution may be followed by a step of eluting with 75% eluting liquid over 20 MV, which may further be followed by a step of eluting with 100% eluting liquid over 20 MV (final NaCl concentration of 1 M). Alternatively to the described elution of a gradient elution optionally followed by one or two steps of elution using eluting liquid having high conductivity, a stepwise elution may be conducted. For instance, a first eluting step, wherein the eluting liquid contains NaCl in a concentration of e.g., 650 mM, and a final second eluting step, wherein the eluting liquid contains NaCl in a concentration of e.g., 1 M, may be conducted over 20 MV for each of both steps.

Finally, with reference to the optional step e) of substituting the eluting liquid by a storage liquid, such storage liquid may comprise, for instance, $MgCl_2$ at about 20 mM, and/or sucrose at about 5%, and may be at a pH of about 7.5.

Integral porous polysaccharide membrane

According to another aspect of the present invention, there is further provided an integral porous polysaccharide membrane which has a first main surface, a second main surface, and membrane bridges which are a solid polymer bulk material surrounding and delimiting the pores of the membrane,

> wherein the average pore diameter of the membrane pores in the porous polysaccharide membrane is from 0.45 to 10.0 $\mu$m,
> wherein the convective porosity of the integral porous polysaccharide membrane is at least 60%,
> wherein the integral porous polysaccharide membrane has chromatographically active centers capable of binding a biomolecule having a hydrodynamic diameter of at least 20 nm attached to its inner and outer surfaces, and
> wherein an amount of the chromatographically active centers per volume of the membrane is from 2 to 12 $\mu$mol/mL and
> an inner surface area of the integral porous polysaccharide membrane is from 1 to 4 $m^2$/g.

The features and (preferred) embodiments described above in the context of the method of purifying biomolecules having a hydrodynamic diameter of at least 20 nm of the present invention also apply for the integral porous polysaccharide membrane of the present invention.

Use of the integral porous polysaccharide membrane

According to the present invention, there is further provided a use of an integral porous polysaccharide membrane as described above for purifying at least one type of biomolecules having a hydrodynamic diameter of at least 20 nm. Preferred uses of said membrane include the purification of lipid double layer-enveloped entities such as liposomes, cell organelles, viruses, virus-like particles (VLPs), extracellular vesicles, and exosomes. Even further preferred is the use in purifying lentiviruses due to their low stability and high sensitivity to increased ionic strength and temperature.

Method of manufacturing an integral porous polysaccharide membrane

In the following, a method of manufacturing the above-described integral porous polysaccharide membrane is described.

Generally, as a starting membrane, a membrane may be manufactured using a known method as described e.g., for a starting material comprised of regenerated cellulose in L. J. Zeman et al., "Microfiltration and ultrafiltration principles and applications", Marcel Dekker 1996, "Part I", "Chapter 3.1", DE 103 26 741 A1, and DE 34 47 625 A1.

In the following, the above method is described in short in relation to a cellulose membrane. A cellulose ester membrane is provided, which may be made of cellulose monoacetate, cellulose diacetate, cellulose triacetate, cellulose propionate, cellulose butyrate, cellulose xanthate, cellulose benzoate, cellulose acetobutyrate, or different suitable cellulose esters, or cellulose nitrate, methylcellulose, ethylcellulose, as well as mixtures thereof, wherein cellulose acetates and particularly cellulose diacetate are preferred. Further, the cellulose ester membrane may be pretreated with additives such as dissolving agents and/or plasticizers. Examples of such additives include acids, e.g., carboxylic acids such as acetic acid, and water-soluble plasticizers for cellulose esters, e.g., diacetin, triacetin, and sulfolane. Then, the cellulose ester membrane is saponified using a saponification medium which is preferably alkaline, e.g., preferable are alkali metal hydroxides. More preferably, an aqueous alcoholic solution of NaOH, KOH, or LiOH is used as saponification medium. Moreover, mixtures of alkali metal hydroxides and other alkaline compounds such as alkali metal carbonates, e.g., $Na_2CO_3$, $K_2CO_3$, $Cs_2CO_3$, or NaHCOs, and/or NasPOa, $K_3PO_4$, sodium silicates, potassium silicates may be used. Saponification may be conducted either using a dry starting material or a wet starting material depending on the pretreatment. With the described method, a flat membrane or a cylindrical membrane (also called hollow fiber membrane, capillary membrane, or tube membrane) can be obtained.

The obtained regenerated cellulose membrane may preferably be exposed to further crosslinking treatment by applying a crosslinker in order to improve chemical strength and/or to introduce functional groups. Said crosslinker has at least two functional moieties being reactive with hydroxyl groups, e.g., as present in cellulose or regenerated cellulose. The crosslinker is not limited and may be selected by a person skilled in the art from known crosslinkers. Preferably, the crosslinker is selected from a diepoxy compound, a diisocyanate compound, epichlorohydrin, epibromohydrin, dimethylurea, dimethylethyleneurea (1,3-dimethyl-2-imidazolidinone), chlorodimethylsilane, bis(2-hydroxyethyl)sulfone, divinyl sulfone, bisacrylamide (N,N'-methylenebisacrylamide), bismaleimides, allyl bromide, an alkyl dihalide compound, an alkylene dihalide compound, a hydroxyalkylene dihalide, a glycidylether compound, and combinations thereof. Suitable functional groups in the crosslinker generally include halides, acrylamides, epoxy groups, isocyanate groups, tosylates, double bonds, triple bonds, and any combination thereof. Preferred glycidylether compounds are 1,4-butanediol diglycidylether, ethylenglycol diglycidylether, glycerin diglycidylether, allyl glycidylether, and polyethyleneglycol diglycidylether. More preferred crosslinkers are 1,4-butanediol diglycidylether and epichlorohydrin. Optionally, a mixture of crosslinkers may also be used. The crosslinking reaction may be conducted in aqueous medium, in an organic solvent, or in a mixture of water and an organic solvent. Preferably, the crosslinking reaction is conducted in aqueous medium. Further, it is preferred to apply a crosslinking catalyst such as NaOH to accelerate the crosslinking reaction. Even further, the crosslinking reaction may be conducted between about 4 °C and the boiling temperature of the medium, wherein a temperature in the range of from 5 °C to 70 °C is preferred and a temperature in the range of from 20 °C to 40 °C is more preferred.

In a specific embodiment of the manufacturing method, the crosslinking reaction may be a so-called "activating crosslinking", i.e., the reaction with e.g., a glycidylether is conducted in a way that a part of the reactive groups e.g., epoxy groups remain unreacted. Thus, in a further step described below, chromatographically active centers/ligands can be bound to said unreacted groups. In the specific case of epoxy groups, such unreacted groups are stable against hydrolysis.

In another specific embodiment of the manufacturing method, the crosslinking method may be a so-called "non-activating crosslinking", i.e., the reaction is conducted in a way that nearly no unreacted groups remain present, i.e., the crosslinking reaction is conducted under harsher conditions (e.g., longer reaction time and/or higher concentration of alkalis and/or higher temperature). Remaining reactive groups may be further deactivated, for instance, epoxy groups may be hydrolyzed using 5% sulfuric acid at elevated temperatures.

Depending on the crosslinker, the concentration of the crosslinker, the concentration of a crosslinking catalyst, the crosslinking duration, optionally the type and concentration of an inert organic solvent, and/or the crosslinking temperature the degree of crosslinking, pore diameter, and the amount of remaining active groups (e.g., epoxy groups) can be adjusted. Thus, an activation, which might be necessary for further processing by binding functional groups, may be conducted directly during the crosslinking.

In a further step, functional groups are introduced to the starting membrane in order to obtain the herein described integral porous polysaccharide membrane. Methods for introducing functional groups are known to a skilled person and are exemplarily described in Greg T. Hermanson, A. Krishna Mallia, Paul K. Smith, Immobilized Affinity Ligand Techniques, Academic Press, INC, 1992. Moreover, methods for grafting functional monomers or polymers to such membranes are known to a skilled person and can also be used to introduce functional groups.

Preferably, functional groups are introduced using epoxy groups or aldehyde groups reacting with hydroxyl groups of the above-described membrane, e.g., a regenerated cellulose membrane. That is, the reaction with epoxy compounds may be conducted during or after the optional crosslinking. Moreover, functional groups may be introduced during the optional crosslinking reaction, e.g., by adding amines and/or monofunctional epoxy compounds, such as phenyl glycidylether or butyl glycidylether, to a diepoxy compound.

In this context, the functional groups are as described above in the context of chromatographically active centers/ligands and include preferably anionic, cationic, hydrophobic, or multi modal groups (i.e., combinations thereof). Further, functional groups may be used which bind biomolecules via electron donor acceptor interactions, such as Lewis acids and bases, via hydrogen bonding, and/or via $\pi$-$\pi$ interactions. In detail, it is referred to the above description of the specific and preferred embodiments of the chromatographically active centers/ligands, which is fully applicable to the method of manufacturing the herein described integral porous polysaccharide membrane.

The Figures show:

Figure 1 shows an exemplary purification of lentiviruses using an inventive D membrane.

Figure 2 shows an exemplary purification of lentiviruses using an inventive Q membrane.

Figure 3 shows an exemplary purification of lentiviruses using a comparative Q membrane.

Figure 4 shows the infective recovery of lentiviruses using an inventive D membrane and comparative D and Q membranes.

The present invention will be further illustrated in the following examples without being limited thereto.

**Examples**

Example 1: Preparation of membranes

a) Precursor membrane

A cellulose acetate membrane was saponified using 15% NaOH in 80/20 ethanol/water for 3 min at ambient temperature. Subsequently, the membrane was washed for 3 min with 6.8% acetic acid in water, twice with ethanol, and 15 min under running reverse osmosis water (ROW) in this order. Then, the membrane was dried for 20 min at 80 °C.

After the above treatment, the saponified and dried membrane was crosslinked using 25% 1,4-butanediol diglycidylether in aqueous 0.1 M NaOH solution followed by incubation of the wet membrane for 22 h at ambient temperature in a closed container. Finally, the incubated membrane was washed for 30 min under running ROW.

b) Q membrane

The above precursor membrane was modified using trimethylamine (10% aqueous solution) for 35 min at 30 °C followed by treatment with 5% sulfuric acid for 5 min at ambient temperature. Then, the membrane was washed for 10 min under running ROW yielding a membrane having quaternary ammonium ligands bound to the membrane (so-called Q membrane).

c) S membrane

The above precursor membrane was modified using an aqueous solution of 30% sodium sulfite and 2.5% $Na_2HPO_4$ at a pH of 8.0 for 45 min at 80 °C followed by washing for 10 min under running ROW, 5 min using 35 g of a 1% HCl solution in ROW, two times 5 min using 30 g of a 1 M NaCl solution in ROW each time, 5 min using 500 g of 5% sulfuric acid, and 10 min under running ROW again. Thus, a membrane having sulfonic acid ligands bound to the membrane (so-called S membrane) is obtained.

d) D membrane

The above precursor membrane was modified using diethylamine (DEA; 10% aqueous solution) for 120 min at 40 °C (or as shown in Table 2 of Example 2) followed by treatment with 5% sulfuric acid for 5 min at ambient temperature. Then, the membrane was washed for 10 min under running ROW yielding a membrane having tertiary ammonium ligands bound to the membrane (so-called D membrane).

e) Comparative membranes: Sartobind Q and Sartobind D

The commercially available membranes Sartobind Q and Sartobind D have been used as comparative membranes (Q and D membrane) having an amount of chromatographically active centers of 120 $\mu$mol/mL and an average pore diameter of 3 to 5 $\mu$m.

f) Adsorption chromatography of lentiviruses using Q and D membranes

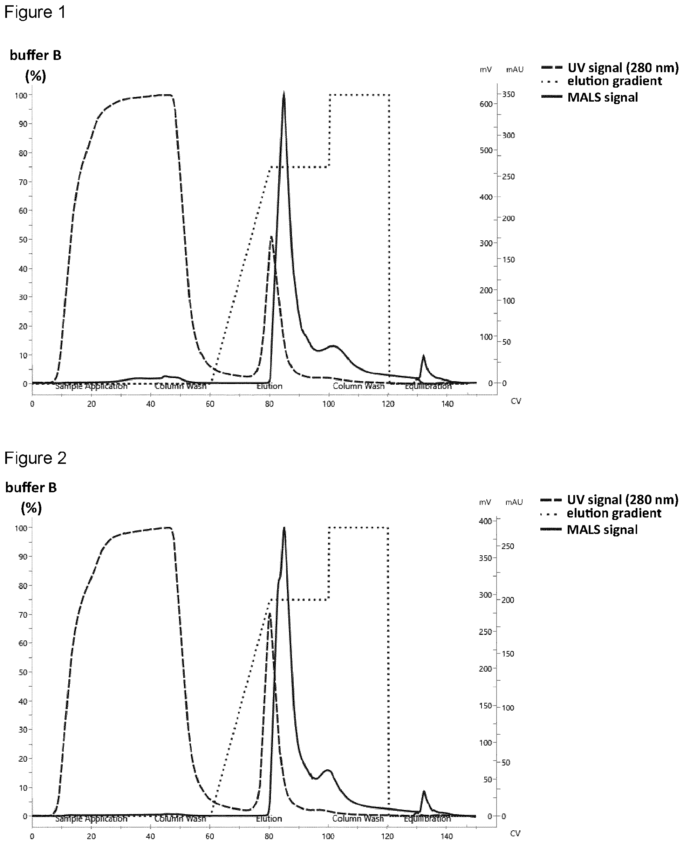

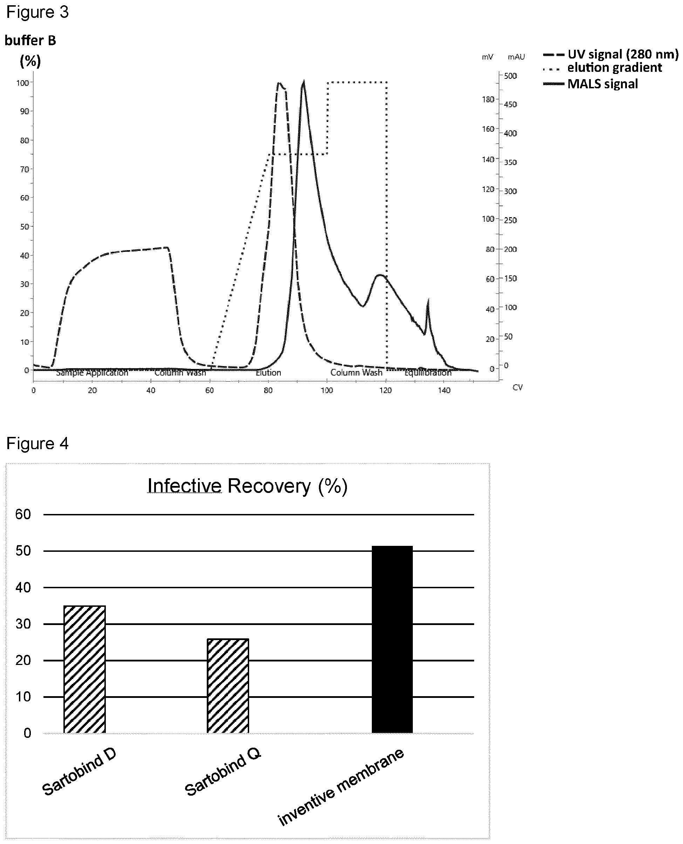

Figure 1 shows an exemplary purification of lentiviruses using an exemplary inventive D membrane (cf. point d) above) and Figure 2 shows an exemplary purification of lentiviruses using an exemplary inventive Q membrane (cf. point b) above). Figure 3 shows a similar exemplary purification using a commercially available Sartobind Q membrane (comparative membrane, cf. point e) above). The purification methods have been conducted using the above-described parameters of pH, conductivity, and temperature (i.e., at a pH of 7.2, a conductivity of the suspension of 7-10 mS/cm, pH of the washing liquid (20 mM Tris-HCl) of 7.2, and pH of the eluting liquid (20 mM Tris-HCl, 1.5 M NaCl for Figure 3; 20 mM Tris-HCl, 0.75 M NaCl for Figures 1 and 2) of 7.2, and at a temperature of 20-25 °C). In this context, the term "CV" relates to "column volumes" as a measure for chromatographic throughput. Moreover, the chromatographic fractions have been analyzed using the UV signal at 280 nm (corresponding to contaminants) and the multi-angle light scattering (MALS) signal (corresponding to lentiviruses), both of which are commonly used and known to a person skilled in the art. For example, a Wyatt Dawn 18 angle light scattering photometer may be used for MALS with a detection at 90° and an irradiation with a 660 nm laser.

Finally, Figure 4 shows the infective recovery of lentiviruses using each of the comparative membranes Sartobind Q and Sartobind D (which are commercially available) as well as an exemplary inventive D membrane. The determination of infective recovery was performed by using an Incucyte® S3 live-cell analysis system as published in Labisch J.J. et al. PLoS One 16, 2021, e0254739.

As can be seen in Figures 1 and 2, the inventive membranes having an average pore diameter of 0.45 to 10.0 $\mu$m, a convective porosity of at least 60%, an amount of chromatographically active centers of 2 to 12 $\mu$mol/mL, and an inner surface area of 1 to 4 m$^2$/g show a low retention of contaminants and a sharp elution of lentiviruses. In contrast, the purification using a Sartobind Q membrane (comparative membrane, cf. Figure 3) shows a large signal of contaminants and a broad and late elution of lentiviruses. That is, the inventive membrane shows a high yield of lentiviruses and selectivity against contaminants as well as yields a highly concentrated purified suspension of lentiviruses.

Moreover, as can be seen from Figure 4, the infective recovery of lentiviruses is significantly increased using the inventive D membrane compared to the comparative membranes Sartobind Q and Sartobind D. That is, the inventive membrane allows mild purification conditions maintaining the structural and functional integrity of the unstable lentiviruses.

Specific properties of exemplary Q membranes according to the present invention are shown in Table 1.

Table 1: Exemplary Q membranes according to the present invention.

| | convective porosity [%] | average pore diameter [$\mu$m] | thickness [$\mu$m] | inner surface area [m$^2$/g] | amount of ligand [$\mu$mol/mL] |
|---|---|---|---|---|---|
| Inv. membrane 1 | 80 | 0.8 | 230 | 3.5 | 6.53 |
| Inv. membrane 2 | 80 | 1.1 | 230 | 3.6 | 6.32 |
| Inv. membrane 3 | 80 | 1.5 | 230 | 2.5 | 3.67 |

Example 2: D membranes having different ligand density

Several D membranes have been prepared as described in Example 1 d) with the specific parameters of the reaction with DEA as shown in Table 2. In this context, each shown value of the membrane thickness, the membrane permeability, and the amount of ligand is the mean of at least 6 measured values.

Table 2: Inventive D membranes having different amounts of ligand.

| | reaction with DEA | | | thickness [μm] | permeability [mD] | amount of ligand [μmol/mL] |
|---|---|---|---|---|---|---|
| | concentration DEA [w%] | reaction temperature [°C] | reaction time [h] | | | |
| Inv. membrane 4 | 8 | 40 | 2.5 | 218 | 102 | 12.2 |
| Inv. membrane 5 | 12 | 35 | 2.5 | 212 | 109 | 11.4 |
| Inv. membrane 6 | 12 | 40 | 2.5 | 214 | 111 | 10.9 |
| Inv. membrane 7 | 8 | 40 | 1.5 | 212 | 110 | 10.5 |
| Inv. membrane 8 | 12 | 35 | 1.5 | 222 | 114 | 10.3 |
| Inv. membrane 9 | 12 | 40 | 1.5 | 213 | 111 | 10.0 |
| Inv. membrane 10 | 10 | 40 | 2.0 | 218 | 113 | 10.8 |
| Inv. membrane 11 | 8 | 35 | 1.5 | 213 | 109 | 9.9 |
| Inv. membrane 12 | 8 | 35 | 2.5 | 221 | 115 | 9.8 |
| Here, Inventive membranes 4 to 12 exhibited a porosity of 80% and an average pore diameter of 1.0 to 1.2 μm. | | | | | | |

**Claims**

1. A method of purifying biomolecules having a hydrodynamic diameter of at least 20 nm, comprising

    a) providing an integral porous polysaccharide membrane and a suspension comprising at least one type of biomolecules having a hydrodynamic diameter of at least 20 nm,

    b) bringing the suspension comprising at least one type of biomolecules into contact with the integral porous polysaccharide membrane to bind the at least one type of biomolecules to the integral porous polysaccharide membrane,

    c) optionally washing the integral porous polysaccharide membrane having bound the at least one type of biomolecules with at least one washing liquid, and

    d) eluting the at least one type of biomolecules from the integral porous polysaccharide membrane by passing an eluting liquid through the integral porous polysaccharide membrane to obtain a purified suspension comprising the at least one type of biomolecules;

    wherein the integral porous polysaccharide membrane has a first main surface, a second main surface, and membrane bridges which are a solid polymer bulk material surrounding and delimiting the pores of the membrane,

    wherein the average pore diameter of the membrane pores in the porous polysaccharide membrane is from 0.45 to 10.0 μm,

    wherein the convective porosity of the integral porous polysaccharide membrane is at least 60%,

    wherein the integral porous polysaccharide membrane has chromatographically active centers capable of binding a biomolecule having a hydrodynamic diameter of at least 20 nm attached to its inner and outer surfaces, and

    wherein an amount of the chromatographically active centers per volume of the membrane is from 2 to 12 μmol/mL and

    an inner surface area of the integral porous polysaccharide membrane is from 1 to 4 m²/g.

2. The method according to claim 1, wherein the at least one type of biomolecules having a hydrodynamic diameter of at least 20 nm are lipid double layer-enveloped entities and wherein the chromatographically active centers are ion-exchange chromatographically active centers.

3. The method according to claim 1 or 2, further comprising a step of gamma-irradiating the integral porous polysaccharide membrane prior to step b) using an irradiation intensity of from 10.0 to 100 kGy.

4. The method according to any one of claims 1 to 3, wherein the pH of the suspension comprising the at least one type of biomolecules is of from 6.0 to 9.0, and/or the pH of the purified suspension comprising the at least one type of biomolecules is of from 6.0 to 9.0.

5. The method according to any one of claims 1 to 4, wherein the conductivity of the suspension of the at least one type of biomolecules is at most 15.0 mS/cm, and/or the conductivity of the washing liquid is of from 5.0 to 15.0 mS/cm, and/or the conductivity of the eluting liquid is at most 85 mS/cm.

6. The method according to any one of claims 1 to 5, wherein the temperature in steps b) to d) is of from 0 to 37 °C.

7. An integral porous polysaccharide membrane which has a first main surface, a second main surface, and membrane bridges which are a solid polymer bulk material surrounding and delimiting the pores of the membrane,

> wherein the average pore diameter of the membrane pores in the porous polysaccharide membrane is from 0.45 to 10.0 $\mu$m,
> wherein the convective porosity of the integral porous polysaccharide membrane is at least 60%,
> wherein the integral porous polysaccharide membrane has chromatographically active centers capable of binding a biomolecule having a hydrodynamic diameter of at least 20 nm attached to its inner and outer surfaces, and
> wherein an amount of the chromatographically active centers per volume of the membrane is from 2 to 12 $\mu$mol/mL and
> an inner surface area of the integral porous polysaccharide membrane is from 1 to 4 $m^2/g$.

8. The integral porous polysaccharide membrane according to claim 7, wherein the polysaccharide is selected from regenerated cellulose, crosslinked cellulose, agarose, and crosslinked agarose.

9. The integral porous polysaccharide membrane according to claim 7 or 8, wherein the chromatographically active centers are anion-exchange chromatographically active centers.

10. The integral porous polysaccharide membrane according to any one of claims 7 to 9, wherein the chromatographically active centers are one or more selected from primary, secondary, tertiary, and quaternary ammonium residues.

11. The integral porous polysaccharide membrane according to any one of claims 7 to 10, wherein the chromatographically active centers are one or more selected from trimethyl ammonium residue, triethyl ammonium residue, dimethyl ammonium residue, diethyl ammonium residue, (2-aminoethyl)ammonium residue, (2-(N,N-diethylamino) ethyl)-N'-ethylammonium residue, (5-amino-3-azapentane)ammonium residue, (2-methylamino ethyl)-N'-methylammonium residue, (2-ethylamino ethyl)-N'-ethylammonium residue, (2-methylamino ethyl)ammonium residue, (3-aminopropyl)ammonium residue, (3-amino-2-hydroxypropyl)ammonium residue, and (3-amino-2-methylpropyl) ammonium residue.

12. The integral porous polysaccharide membrane according to any one of claims 7 to 11, wherein a thickness of the integral porous polysaccharide membrane is from 50 to 1000 $\mu$m.

13. The integral porous polysaccharide membrane according to any one of claims 7 to 12, further comprising a support structure.

14. The integral porous polysaccharide membrane according to any one of claims 7 to 13, wherein the average pore diameter of the membrane pores in the porous polysaccharide membrane is from 0.45 to 10.0 $\mu$m,

> wherein the thickness of the porous polysaccharide membrane is from 150 to 300 $\mu$m, and
> wherein the convective porosity of the porous polysaccharide membrane is at least 65%.

15. Use of an integral porous polysaccharide membrane according to any one of claims 7 to 14 for purifying at least one type of biomolecules having a hydrodynamic diameter of at least 20 nm.

Figure 1

Figure 2

Figure 3

Figure 4

Europäisches
Patentamt
European
Patent Office
Office européen
des brevets

# EUROPEAN SEARCH REPORT

**Application Number**

EP 24 17 6330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/099522 A1 (MERCK MILLIPORE LTD [IE]) 27 May 2021 (2021-05-27)<br>* claim 1 *<br>* figure 1b *<br>* claims 50, 56-57 *<br>* page 15, line 27 - line 30 *<br>* claim 17 *<br>* page 14, line 1 - line 26 *<br>* page 16, line 21 - line 25 *<br>----- | 7-14 | INV.<br>B01D15/32<br>B01D15/36<br>B01D15/38<br>B01D67/00<br>B01D69/02<br>C07K1/18<br>C12N7/00 |
| X | WO 2023/287718 A1 (DONALDSON CO INC [US]) 19 January 2023 (2023-01-19)<br>* claim 1 *<br>* claim 5 *<br>* paragraph [0054] *<br>* claim 7 *<br>* claim 4 *<br>* claim 6 *<br>* paragraph [0046] *<br>----- | 7,8,12, 13 | |
| X | WO 2004/073843 A1 (UNIV MCMASTER [CA]; CHILDS RONALD F [CA] ET AL.) 2 September 2004 (2004-09-02) | 1,2,4-6, 15 | |
| Y | * claim 38 *<br>* claim 39 *<br>* claim 70 *<br>* claim 15 *<br>* claims 6,7 *<br>* claim 12 *<br>* page 51, line 21 - line 22 *<br>* claims 21,22 *<br>* figures 19A, 19B *<br>----- | 3 | |
| Y | US 2022/241484 A1 (SWARTJES JAN [DE] ET AL) 4 August 2022 (2022-08-04)<br>* paragraph [0174]; claims 9, 13 *<br>* paragraph [0135] *<br>----- | 3 | |

**TECHNICAL FIELDS SEARCHED (IPC)**

B01D
C07K
C12N

The present search report has been drawn up for all claims

| Place of search | Date of completion of the search | Examiner |
|---|---|---|
| Munich | 30 October 2024 | Oikonomou, Evdokia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................................
& : member of the same patent family, corresponding
    document

EPO FORM 1503 03.82 (P04C01)

**EP 4 650 025 A1**

**ANNEX TO THE EUROPEAN SEARCH REPORT
ON EUROPEAN PATENT APPLICATION NO.**               EP 24 17 6330

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2024

| Patent document cited in search report | | Publication date | Patent family member(s) | | Publication date |
|---|---|---|---|---|---|
| WO 2021099522 | A1 | 27-05-2021 | AU | 2020387638 A1 | 02-06-2022 |
| | | | CA | 3161993 A1 | 27-05-2021 |
| | | | CN | 115066288 A | 16-09-2022 |
| | | | EP | 4061515 A1 | 28-09-2022 |
| | | | JP | 2023502481 A | 24-01-2023 |
| | | | KR | 20220119035 A | 26-08-2022 |
| | | | TW | 202135921 A | 01-10-2021 |
| | | | US | 2022410076 A1 | 29-12-2022 |
| | | | WO | 2021099522 A1 | 27-05-2021 |
| WO 2023287718 | A1 | 19-01-2023 | EP | 4370227 A1 | 22-05-2024 |
| | | | JP | 2024533931 A | 18-09-2024 |
| | | | WO | 2023287718 A1 | 19-01-2023 |
| WO 2004073843 | A1 | 02-09-2004 | AT | E448866 T1 | 15-12-2009 |
| | | | AU | 2004212641 A1 | 02-09-2004 |
| | | | AU | 2009230738 A1 | 12-11-2009 |
| | | | CA | 2514471 A1 | 02-09-2004 |
| | | | EP | 1617936 A1 | 25-01-2006 |
| | | | EP | 2143481 A1 | 13-01-2010 |
| | | | EP | 2143482 A1 | 13-01-2010 |
| | | | JP | 5189286 B2 | 24-04-2013 |
| | | | JP | 5432934 B2 | 05-03-2014 |
| | | | JP | 2006519273 A | 24-08-2006 |
| | | | JP | 2011149024 A | 04-08-2011 |
| | | | KR | 20050107440 A | 11-11-2005 |
| | | | US | 2004203149 A1 | 14-10-2004 |
| | | | US | 2008312416 A1 | 18-12-2008 |
| | | | US | 2008314831 A1 | 25-12-2008 |
| | | | US | 2009008328 A1 | 08-01-2009 |
| | | | US | 2009029438 A1 | 29-01-2009 |
| | | | US | 2009032463 A1 | 05-02-2009 |
| | | | US | 2009035552 A1 | 05-02-2009 |
| | | | US | 2010044316 A1 | 25-02-2010 |
| | | | US | 2010047551 A1 | 25-02-2010 |
| | | | WO | 2004073843 A1 | 02-09-2004 |
| US 2022241484 | A1 | 04-08-2022 | AU | 2020231463 A1 | 23-09-2021 |
| | | | CA | 3130651 A1 | 10-09-2020 |
| | | | CN | 113543821 A | 22-10-2021 |
| | | | EP | 3934712 A1 | 12-01-2022 |
| | | | JP | 7526737 B2 | 01-08-2024 |
| | | | JP | 2022524021 A | 27-04-2022 |
| | | | KR | 20210136035 A | 16-11-2021 |
| | | | US | 2022241484 A1 | 04-08-2022 |
| | | | WO | 2020178420 A1 | 10-09-2020 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 17 6330

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2024

| Patent document cited in search report | Publication date | Patent family member(s) | Publication date |
|---|---|---|---|
| | | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009127285 A1
- DE 10326741 A1
- DE 3447625 A1

**Non-patent literature cited in the description**

- *Journal of Chromatography A - J CHROMATOGR A.*, vol. 731, 27-40
- **ATKINS**. Physikalische Chemie, 934-936
- **LEY et al.** *Journal of Membrane Science*, 2018, vol. 564, 543-551
- **L. J. ZEMAN et al.** Microfiltration and ultrafiltration principles and applications. Marcel Dekker, 1996
- **GREG T. HERMANSON** ; **A. KRISHNA MALLIA** ; **PAUL K. SMITH**. Immobilized Affinity Ligand Techniques. Academic Press, INC, 1992
- **LABISCH J.J. et al.** *PLoS One*, 2021, vol. 16, e0254739